(19) Europäisches Patentamt
European Patent Office
Office européen des brevets

(11) Veröffentlichungsnummer: **0 258 855 B1**

(12) **EUROPÄISCHE PATENTSCHRIFT**

(45) Veröffentlichungstag der Patentschrift: **12.02.92**

(51) Int. Cl.5: **C07D 333/38**

(21) Anmeldenummer: **87112628.0**

(22) Anmeldetag: **29.08.87**

# (54) Verfahren zur Herstellung von Aminothiophenderivaten.

(30) Priorität: **04.09.86 DE 3630070**

(43) Veröffentlichungstag der Anmeldung:
**09.03.88 Patentblatt 88/10**

(45) Bekanntmachung des Hinweises auf die Patenterteilung:
**12.02.92 Patentblatt 92/07**

(84) Benannte Vertragsstaaten:
**CH DE ES FR GB IT LI NL**

(56) Entgegenhaltungen:
**EP-A- 0 193 885**
**DE-A- 3 507 421**

**CHEMICAL ABSTRACTS, Band 104, Nr. 3, 20. Januar 1986, Seite 476, Zusammenfassung Nr. 19505f, Columbus, Ohio, US; & JP-A-60 161 978 (MITSUI TOATSU CHEMICALS, INC.) 23-08-1985**

**CHEMICAL ABSTRACTS, Band 104, Nr. 11, 17. März 1986, Seite 646, Zusammenfassung Nr. 88420m, Columbus, Ohio, US; & JP-A-60 190 776 (MITSUI TOATSU CHEMICALS, INC.) 28-09-1985**

**I.Org. Chem. 38, 3615-3617 (1973)**

**Heterocycl. Chem. 16, 1541-1543 (1979)**

(73) Patentinhaber: **BASF Aktiengesellschaft**
**Carl-Bosch-Strasse 38**
**W-6700 Ludwigshafen(DE)**

(72) Erfinder: **Etzbach, Karl-Heinz, Dr.**
**Carl-Bosch-Ring 55**
**W-6710 Frankenthal(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung betrifft ein Verfahren zur Herstellung von Aminothiophenderivaten der Formel I

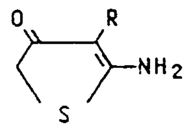

(I),

in der

R $C_1$-$C_8$-Alkoxycarbonyl, dessen Alkylkette gegebenenfalls durch ein oder mehrere Sauerstoffatome unterbrochen ist, Carbamoyl, $C_1$-$C_4$-Mono-oder Dialkylcarbamoyl, das gegebenenfalls durch Hydroxy oder $C_1$-$C_4$-Alkoxy substituiert ist, Phenylcarbamoyl oder Cyano bedeuten,

das dadurch gekennzeichnet ist, daß man Verbindungen der Formel II

$NC$-$CH_2$-$R$ (II),

in der R die obengenannte Bedeutung besitzt, in Wasser mit einer Base und gleichzeitig oder anschließend mit einem Halogenacetylhalogenid umsetzt und danach mit Schwefelwasserstoff oder einem wasserlöslichen Sulfid reagieren läßt.

Im Hinblick auf die ältere Anmeldung EP-A- 193 885 (benannte Vertragsstaaten: BE, CH, DE, FR, GB, IT, LI und NL) , in der in Beispiel 4 die Herstellung von 2-Amino-3-cyano-4-hydroxythiophen durch Umsetzung von Chloracetylchlorid mit Malodinitril in N,N-Dimethylformamid in Gegenwart von Triethylamin und anschließender Behandlung des Reaktionsgemisches mit wäßriger Ammoniumsulfidlösung beschrieben ist, wurden für die Vertragsstaaten CH, DE, FR, GB, IT, LI und NL gesonderte Patentansprüche vorgelegt.

Weiterhin wird in Chem. Abstracts, Band 104, 19 505 f und 88 420 m beschrieben, daß man durch Umsetzung von Thioglykolsäure oder deren Estern mit Malodinitril in Gegenwart einer Base (z.B. Natriumethanolat) in Ethanol als Lösungsmittel zu 2-Amino-3-cyano-4-hydroxythiophen gelangen würde.

In Wirklichkeit entsteht unter den genannten Bedingungen kein Aminothiophenderivat, sondern eine Thiazolverbindung. Dies geht aus Untersuchungen hervor, die in J. Org. Chem., Band 38, Seiten 3615 bis 3617, 1973, und J. Heterocycl. Chem., Band 16, Seiten 1541 bis 1543, 1979, veröffentlicht sind.

Alle in den Formeln I und II auftretenden Alkylgruppen können sowohl geradkettig als auch verzweigt sein.

Reste R sind z.B. $COOCH_3$, $COOC_2H_5$, $COO_3H_7$, $COOC_4H_9$, $COOC_5H_{11}$, $COOC_6H_{15}$, $COOC_7H_{15}$, $COOC_8H_{17}$,

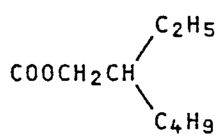

,

$COOCH_2CH_2OCH_3$, $COOC_2H_4OC_2H_5$, $CONH_2$, $CONHCH_3$, $CONHC_2H_5$, $CONHC_3H_7$, $CONHC_4H_9$, $CONHC_6H_5$, $CONHC_2H_4OH$, $CONHC_2H_4OCH_3$, $CON(CH_3)_2$, $CON(C_2H_5)_2$, $CON(C_3H_7)_2$, $CON(C_4H_9)_2$ oder $CON(C_2H_4OCH_3)_2$. Vorzugsweise steht R für Cyano.

Für die Umsetzung geeignete Basen sind z.B. Amine oder Hydroxide, Carbonate oder Hydrogencarbonate von Alkali- oder Erdalkalimetallen.

Einzelne Basen sind beispielsweise Trimethyl, Triethyl, Tripropyl, Tributyl, Tri(2-hydroxyethyl)- oder Tri (2-hydroxypropyl)-amin, Pyridin, Magnesium-, Calcium-, Strontium-, Barium-, Lithium-, Natrium- oder Kaliumhydroxid, Lithium-, Natrium- oder Kaliumcarbonat oder Lithium-, Natrium- oder Kaliumhydrogencarbonat.

Besonders bevorzugt sind dabei die Carbonate, insbesondere Kaliumcarbonat.

Halogenacetylhalogenide sind insbesondere die Chlor- oder Bromverbindungen, wobei aus wirtschaftlichen Gründen Chloracetylchlorid bevorzugt ist.

Als lösliche Sulfide sind insbesondere Alkalisulfide, Ammoniumsulfid oder die entsprechenden Hydrogensulfide zu nennen. Vorzugsweise verwendet man Schwefelwasserstoff, Ammoniumsulfid oder Natrium- oder Kaliumhydrogensulfid.

Das erfindungsgemäße Verfahren wird zweckmäßig so vorgenommen, daß man 2 Mol der Base,

bezogen auf ein Mol der Verbindung der Formel II, in Wasser vorlegt, dann die Verbindung II und gegebenenfalls gleichzeitig oder vorzugsweise anschließend Chloracetylchlorid langsam zugibt. Man verwendet dabei mindestens 1 Mol Chloracetylchlorid, vorzugsweise aber einen Überschuß, jeweils bezogen auf ein Mol der Verbindung der Formel II.

Im erfindungsgemäßen Verfahren liegt die Reaktionstemperatur vorzugsweise zwischen 0 und 20 °C. Der Reaktionsmischung können auch inerte Lösungsmittel, wie N,N-Dimethylformamid, N,-Methylpyrrolidon, Methanol, Ethanol, Isopropanol, Butanol, Butylglykol, Chloroform, Methylenchlorid, 1,1,1-Trichlorethan, 1,1,2-Trichlorethan, Toluol, Xylol, Chlorbenzol, Nitrobenzol, Essigsäureethylester oder Diethylether, sowie Phasentransferkatalysatoren, wie Tetrabutylammoniumbromid, Tricaprylmethylammoniumchlorid, Benzyltrimethylammoniumchlorid, Benzyltriethylammoniumchlorid, (2-Hydroxyethyl)-triethylammoniumchlorid oder Hexadecyl-tributylphosphoniumbromid zugesetzt werden. Dies ist jedoch nicht erforderlich.

Die Umsetzung mit der Schwefelverbindung wird anschließend an die erste Umsetzung bei einer Temperatur von ungefähr 0 bis 40 °C vorgenommen, wobei die äquivalente Menge oder ein Überschuß an Schwefelverbindung, jeweils bezogen auf die Verbindung II, verwendet wird. Die Aminothiophenderivate fallen in der Regel aus der Reaktionsmischung aus und können nach an sich bekannten Methoden isoliert werden.

Man erhält die Aminothiophenderivate der Formel I nach dem erfindungsgemäßen Verfahren in guter Ausbeute und Reinheit. Sie dienen als Vorprodukte, z.B. für Diazokomponenten der Formel III

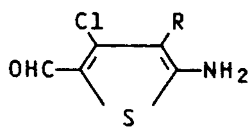

(III),

in der R die obengenannte Bedeutung besitzt, mit denen sich wertvolle Dispersionsfarbstoffe herstellen lassen (s. z.B. DE-A-3 529 831 oder DE-A-3 517 365).

Die folgenden Beispiele sollen die Erfindung näher erläutern. In ihnen beziehen sich Angaben über Teile und Prozente, sofern nicht anders vermerkt, auf das Gewicht.

Beispiel 1

Zu einem Gemisch aus 250 Teilen Wasser, 250 Teilen Eis und 152 Teilen Kaliumcarbonat gab man 33 Teile Malondinitril. Die Mischung wurde 1 Stunde bei 0 °C gerührt, dann tropfte man unter Eiskühlung 67,4 Teile Chloracetylchlorid zu und ließ noch 2 Stunden bei 0 °C rühren. Anschließend leitete man in die Suspension unter Rühren bei 0 °C 18,7 Teile Schwefelwasserstoff ein, ließ die Mischung auf Raumtemperatur kommen, rührte noch 2 Stunden bei Raumtemperatur nach und saugte den Niederschlag ab. Das Produkt wurde noch mit Wasser gewaschen und bei 70 °C im Umluftschrank getrocknet.

Man erhielt 31 Teile (44 % d.Th.) 2-Amino-3-cyano-4-hydroxythiophen Schm. >300 °C, IR (KBr): 3260, 3061 ($NH_2$), 2219 (C≡N), 1668, 1641 $cm^{-1}$ (C=0).

Beispiel 2

Zu einem Gemisch aus 250 Teilen Wasser, 250 Teilen Eis und 152 Teilen Kaliumcarbonat gab man eine Lösung aus 33 Teilen Malondinitril in 100 Teilen N,N-Dimethylformamid. Anschließend verfuhr man wie in Beispiel 1 beschrieben.

Man erhielt 48 Teile (69 % d.Th.) 2-Amino-3-cyano-4-hydroxythiophen.

Beispiel 3

Zu einer Mischung aus 250 Teilen Methylenchlorid, 500 Teilen Wasser und 138 Teilen Kaliumcarbonat gab man bei 0 °C 33 Teile Malondinitril und rührte 1 Stunde bei 0 °C nach. Dann tropfte man unter Eiskühlung 56,6 Teile Chloracetylchlorid zu und ließ noch 2 Stunden bei 0 °C rühren. Anschließend leitete man unter Rühren und Eiskühlung 18,7 Teile Schwefelwasserstoff ein, ließ die Mischung auf Raumtemperatur kommen, rührte noch 2 Stunden bei Raumtemperatur nach und saugte den Niederschlag ab. Das Produkt wurde mit Wasser gewaschen und bei 70 °C im Umluftschrank getrocknet. Man erhielt 44 Teile (63 % d.Th.) 2-Amino-3-cyano-4-hydroxythiophen.

Beispiel 4

Man verfuhr wie in Beispiel 3 beschrieben, gab aber zusätzlich zum Kaliumcarbonat noch 3 Teile Tricaprylmethylammoniumchlorid zu.

Man erhielt 51 Teile (73 % d.Th.) 2-Amino-3-cyano-4-hydroxythiophen.

**Patentansprüche**

**Patentansprüche für folgende Vertragsstaaten : CH, DE, FR, GB, IT, LI, NL**

**1.** Verfahren zur Herstellung von Aminothiophenderivaten der Formel I

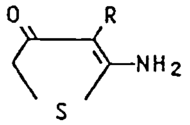

(I),

in der

R $C_1$-$C_8$-Alkoxycarbonyl, dessen Alkylkette gegebenenfalls durch ein oder mehrere Sauerstoffatome unterbrochen ist, Carbamoyl, $C_1$-$C_4$-Mono-oder Dialkylcarbamoyl, das gegebenenfalls durch Hydroxy oder $C_1$-$C_4$-Alkoxy substituiert ist oder Phenylcarbamoyl bedeuten,

dadurch gekennzeichnet, daß man Verbindungen der Formel II

$NC-CH_2-R$ (II),

in der R die obengenannte Bedeutung besitzt, in Wasser mit einer Base und gleichzeitig oder anschließend mit einem Halogenacetylhalogenid umsetzt und danach mit Schwefelwasserstoff oder einem wasserlöslichen Sulfid reagieren läßt.

**2.** Verfahren zur Herstellung des Aminothiophenderivats der Formel Ia

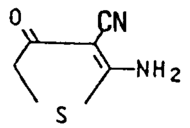

(Ia)

durch Umsetzung von Malondinitril in einem Reaktionsmedium mit einer Base und gleichzeitig oder anschließend mit einem Halogenacetylhalogenid und daran anschließender Reaktion mit Schwefelwasserstoff oder einem wasserlöslichen Sulfid, dadurch gekennzeichnet, daß man von Anfang an Wasser als Reaktionsmedium verwendet.

**3.** Verfahren nach Anspruch 1 oder 2, dadurch gekennzeichnet, daß man die Umsetzung mit Halogenacetylhalogenid bei einer Temperatur Von 0 bis 20 °C durchführt.

**4.** Verfahren nach Anspruch 1 oder 2, dadurch gekennzeichnet, daß man als Base ein Carbonat verwendet.

**5.** Verfahren nach Anspruch 1 oder 2, dadurch gekennzeichnet, daß man mindestens 2 Mol Base und mindestens 1 Mol Halogenacetylhalogenid, jeweils bezogen auf ein Mol der Verbindung der Formel II, verwendet.

**Patentansprüche für folgenden Vertragsstaat : ES**

**1.** Verfahren zur Herstellung von Aminothiophenderivaten der Formel I

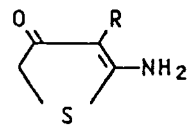

(I),

in der

R $C_1$-$C_8$-Alkoxycarbonyl, dessen Alkylkette gegebenenfalls durch ein oder mehrere Sauerstoffatome unterbrochen ist, Carbamoyl, $C_1$-$C_4$-Mono-oder Dialkylcarbamoyl, das gegebenenfalls durch Hydroxy oder $C_1$-$C_4$-Alkoxy substituiert ist, Phenylcarbamoyl oder Cyano bedeuten,

dadurch gekennzeichnet, daß man Verbindungen der Formel II

$NC-CH_2-R$ (II),

in der R die obengenannte Bedeutung besitzt, in Wasser mit einer Base und gleichzeitig oder anschließend mit einem Halogenacetylhalogenid umsetzt und danach mit Schwefelwasserstoff oder einem wasserlöslichen Sulfid reagieren läßt.

2. Verfahren gemäß Anspruch 1, dadurch gekennzeichnet, daß man als Verbindung II Malondinitril verwendet.

3. Verfahren gemäß Anspruch 1, dadurch gekennzeichnet, daß man die Umsetzung mit Halogenacetylhalogenid bei einer Temperatur von 0 bis 20 °C durchführt.

4. Verfahren gemäß Anspruch 1, dadurch gekennzeichnet, daß man als Base ein Carbonat verwendet.

5. Verfahren gemäß Anspruch 1, dadurch gekennzeichnet, daß man mindestens 2 Mol Base und mindestens 1 Mol Halogenacetylhalogenid, jeweils bezogen auf ein Mol der Verbindung der Formel II, verwendet.

**Claims**

**Claims for the following Contracting States : CH, DE, FR, GB, IT, LI, NL**

1. A process for the preparation of an aminothiophene derivative of the formula I

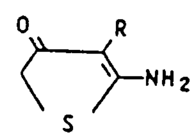

(I)

where R is $C_1$-$C_8$-alkoxycarbonyl whose alkyl chain may be interrupted by one or more oxygen atoms, carbamyl, $C_1$-$C_4$-mono- or dialkylcarbamyl which is unsubstitute or substituted by hydroxyl or $C_1$-$C_4$-alkoxy, or phenylcarbamyl, wherein a compound of the formula II

$NC-CH_2-R$ (II),

where R has the above meanings, is reacted in water with a base and simultaneously or subsequently with a haloacetyl halide, and the product is then reacted with hydrogen sulfide or a water-soluble sulfide.

2. A process for the preparation of an aminothiophene derivative of the formula Ia

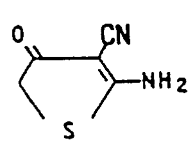

(Ia)

by reacting malonodinitrile in a reaction medium with a base and then with a haloacetyl halide and thereafter reacting the product with hydrogen disulfide or with a water-soluble sulfide, when water is used as the reaction medium from the outset.

**3.** A process as claimed in claim 1 or 2, wherein the reaction with the haloacetyl halide is carried out at from 0 to 20 °C.

**4.** A process as claimed in claim 1 or 2, wherein the base used is carbonate.

**5.** A process as claimed in claim 1 or 2, wherein not less than 2 moles of base and not less than 1 mole of haloacetyl halide are used per mole of a compound of the formula II.

**Claims for the following Contracting State : ES**

**1.** A process for the preparation of an aminothiophene derivative of the formula I

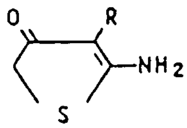

(I)

where R is $C_1$-$C_8$-alkoxycarbonyl whose alkyl chain may be interrupted by one or more oxygen atoms, carbamyl, $C_1$-$C_4$-mono- or dialkylcarbamyl which is unsubstituted or substituted by hydroxyl or $C_1$-$C_4$-alkoxy, or phenylcarbamyl or cyano, wherein a compound of the formula II

$NC$-$CH_2$-$R$ (II),

where R has the above meanings, is reacted in water with a base and simultaneously or subsequently with a haloacetyl halide, and the product is then reacted with hydrogen sulfide or a water-soluble sulfide.

**2.** A process as claimed in claim 1, wherein malodinitrile is used as compound II.

**3.** A process as claimed in claim, wherein the reaction with the haloacetyl halide is carried out at from 0 to 20 °C.

**4.** A process as claimed in claim, wherein the base used is carbonate.

**5.** A process as claimed in claim 1, wherein not less than 2 moles of base and not less than 1 mole of haloacetyl halide are used per mole of a compound of the formula II.

**Revendications**

**Revendications pour les Etats contractants suivants : CH, DE, FR, GB, IT, LI, NL**

**1.** Procédé de préparation de dérivés d'aminothiophène de formule I

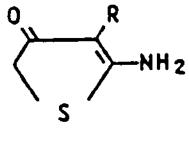

(I),

dans laquelle R représente un groupement (alcoxy en $C_1$-$C_8$) carbonyle dont la chaîne alkyle est éventuellement interrompue par un ou plusieurs atomes d'oxygène, carbamoyle, mono ou di(alkyl en $C_1$-$C_4$)carbamoyle qui est éventuellement substitué par un groupement hydroxy ou par un groupement alcoxy en $C_1$-$C_4$, ou phénylcarbamoyle, caractérisé en ce qu'on fait réagir des composés de formule II

NC-$CH_2$-R (II),

dans laquelle R a la signification donnée précédemment, dans de l'eau avec une base et en même temps ou après avec un halogénure d'halogénoacétyle puis on fait réagir avec de l'hydrogène sulfuré ou un sulfure soluble dans l'eau.

**2.** Procédé de préparation du dérivé d'aminothiophène de formule Ia

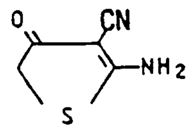

(Ia)

par réaction du malodinitrile dans un milieu de réaction avec une base et en même temps ou après avec un halogénure d'halogénoacétyle et ensuite par réaction avec de l'hydrogène sulfuré ou un sulfure soluble dans l'eau, caractérisé en ce que dès le début on utilise de l'eau comme milieu réactionnel.

**3.** Procédé selon la revendication 1 ou 2, caractérisé en ce qu'on effectue la réaction avec l'halogénure d'halogénoacétyle à une température de 0 à 20 °C.

**4.** Procédé selon la revendication 1 ou 2, caractérisé en ce qu'on utilise un carbonate en tant que base.

**5.** Procédé selon la revendication 1 ou 2, caractérisé en ce qu'on utilise au moins 2 moles de base et au moins 1 mole d'halogénure d'halogénoacétyle, à chaque fois pour une mole du composé de formule II.

**Revendications pour l'Etat contractant suivant : ES**

**1.** Procédé de préparation de dérivés d'aminothiophène de formule I

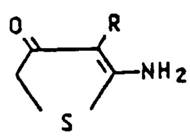

(I),

dans laquelle R est un groupement (alcoxy en $C_1$-$C_8$)carbonyle dont la chaîne alkyle est éventuellement interrompue par un ou plusieurs atomes d'oxygène, carbamoyle, mono ou di(alkyle en $C_1$-$C_4$)-carbamoyle qui est éventuellement substitué par un groupement hydroxy ou un groupement alcoxy en $C_1$-$C_4$, phénylcarbamoyle ou cyano,
caractérisé en ce qu'on fait réagir des composés de formule II

NC-$CH_2$-R (II),

dans laquelle R a la signification donnée ci-dessus, dans de l'eau avec une base et en même temps ou après avec un halogénure d'halogénoacétyle puis on fait réagir avec de l'hydrogène sulfuré ou un sulfure soluble dans l'eau.

**2.** Procédé selon la revendication 1, caractérisé en ce qu'on utilise comme composé II du malodinitrile.

**3.** Procédé selon la revendication 1, caractérisé en ce qu'on effectue la réaction avec l'halogénure d'halogénoacétyle a une température de 0 a 20 °C.

**4.** Procédé selon la revendication 1, caractérisé en ce qu'on utilise un carbonate en tant que base.

**5.** Procédé selon la revendication 1, caractérisé en ce qu'on utilise au moins 2 moles de base et au moins 1 mole d'halogénure d'halogénoacétyle, à chaque fois pour une mole du composé de formule II.